# EUROPEAN PATENT APPLICATION

(11) **EP 0 965 372 A2**
(43) Date of publication of application: **22.12.1999**
(21) Application number: 99108709.9
(22) Date of filing: 30.04.1999
(51) Int. Cl.: B01D 1/00, A61M 16/18

(54) **Vaporiser**

(30) Priority: 15.06.1998 SE 9802124
(71) Applicant: Siemens-Elema AB, 171 95 Solna 1 (SE)
(72) Inventor: Castor, Rolf, 12942 Hägersten (SE); Emtell, Pär, 16247 Vällingby (SE); Pessala, Tom, 16834 Bromma (SE)

(57) **Abstract**

A vaporiser comprises a vaporising chamber (1) for vaporising a liquid additive (3) such as a liquid anaesthetic and in which are disposed a carrier gas inlet (8) and a carrier gas outlet (9) co-operatively arranged to define opposite ends of a carrier gas flow path (12) which path is communicable with vapour of the vaporising chamber (1). An element (5) of porous material, such as a microporous membrane (7), having pores sized to exclude passage therethrough of the liquid additive (3) is disposed in the carrier gas flow path (12) to act as a barrier to the egress of the liquid additive (3)from the vaporiser (1).

## Description

The present invention relates to a vaporiser for a liquid additive and in particular to a vaporiser in which vapour of a volatile liquid additive such as an anaesthetic or analgesic is mixed with a carrier gas (which term is to be understood to include gas mixtures) before delivery to a patient.

Vaporisers may be used in a variety of applications but for ease of reference in the following description the invention will be described in relation to medical vaporisers.

Such vaporisers generally operate by vaporising a volatile liquid anaesthetic and mixing the vapour with a carrier gas which may then introduced into the breathing circuit of a patient anaesthetic machine for inhalation by a patient.

"Through-flow" type vaporisers are known in which a carrier gas is passed from an inlet, through a vaporising chamber to in order to collect the vapour. It is known to provide a vaporiser having a vaporising chamber comprising a liquid holding portion and a vapour receiving portion a carrier gas inlet and an outlet is disposed within the vaporising chamber to define a carrier gas flow path. In one known type of vaporiser the inlet is provided in the form of a capillary passage disposed within in the liquid holding portion of the vaporisation chamber. Carrier gas may then be and bubbled through liquid anaesthetic which causes the liquid to vaporise as the bubbles burst at the liquid surface proximal the vapour receiving portion of the vaporising chamber. A mixture of vapour and carrier gas may then pass through the outlet which is disposed within the vapour receiving portion of the chamber. However, only a relatively small number of such passages may be economically provided within the chamber and they typically project into the chamber so that such "bubble-type" vaporisers are relatively inefficient.

Additionally, the flow of carrier gas through the liquid has to be carefully regulated in order to maintain the correct dosage of anaesthetic vapour as the volume of the vapour receiving portion increases as the liquid vaporises.

A flow-through type anaesthetic vaporiser having a substantially constant volume of liquid is disclosed in GB 2 279 015 A and comprises a vaporisation chamber having a carrier gas inlet and a carrier gas outlet arranged within the chamber so that together they define a flow path for the carrier gas through liquid anaesthetic held within a wick material in the chamber. The wick material is in contact with a liquid reservoir which replenishes vaporised liquid and thus maintains a substantially constant liquid volume for vaporisation. However using this wick material increases the overall size of the vaporiser.

In both cases a further disadvantage is that liquid droplets may also pass out of the vaporisation chamber with the carrier gas. These may adversely affect the anaesthetic dose administered to the patient.

A further known anaesthetic vaporiser is disclosed in US 5 235 971 and comprises a vaporising chamber that is configured with a liquid holding portion and a vapour holding portion above the liquid holding portion and a heater to vaporise the liquid. A carrier gas inlet and outlet are arranged within the vaporiser to define a flow path that lies completely outside the vaporisation chamber. A delivery channel is provided between the vapour within the chamber and the carrier gas flow path and a valve arrangement is provided within the channel, between the chamber and the carrier gas flow path to regulate the delivery of vapour to the carrier gas. Again problems may still occur with liquid droplets if for example vapour is allowed to condense in the valve arrangement.

It is the aim of the present invention to provide a vaporiser in which at least some of the problems of the prior art devices are alleviated.

This is achieved by the invention according to and characterised by claim 1 in which a porous element is disposed within a vaporisation chamber to act as a barrier to the egress of the liquid additive such as a liquid anaesthetic, which may for example, be the common anaesthetics halothane, enflurane, isoflurane, sevoflurane or desflurane.

Usefully, the porous element may be disposed to prevent egress of liquid additive through the outlet of the vaporiser. Particularly usefully the element may be arranged to also act as a base wall of the vaporisation chamber to support the liquid additive in a liquid holding portion above a vapour receiving portion of the chamber. The inlet and the outlet for the carrier gas may be arranged so that carrier gas flows exclusively within the vapour receiving portion to collect vapour that has diffused through the porous element. Since the vapour receiving portion is below the liquid holding portion then vaporisation is substantially unaffected by changes in the volume of liquid as it evaporates.

Alternatively, the porous element may be arranged to act as a base wall of the vaporisation chamber to support the liquid additive in a liquid holding portion thereof above the carrier gas inlet with the outlet disposed within a vapour receiving portion of the chamber above the liquid additive. In this way the bubbles may be conveniently and efficiently generated across the extent of the element, which may form the entire base wall, at the bottom of the liquid so that bubbles pass through substantially all of the liquid volume. An additional porous element may be advantageously disposed before the outlet to prevent the passage of any liquid droplets out of the vaporiser.

Conveniently, the porous element may comprise a microporous membrane disposed over a rigid grating.

Embodiments of the invention will now be described, by way of example only, with reference to the drawings of the accompanying figures of which:
Figure 1 shows a representation of a first embodiment of a vaporiser according to the present invention.
Figure 2 shows a representation of a second embodiment according to the present invention.
Figure 3 shows a modification to the vaporiser shown in Figure 2.
Figure 4 shows a schematic representation of a third embodiment according to the present invention.

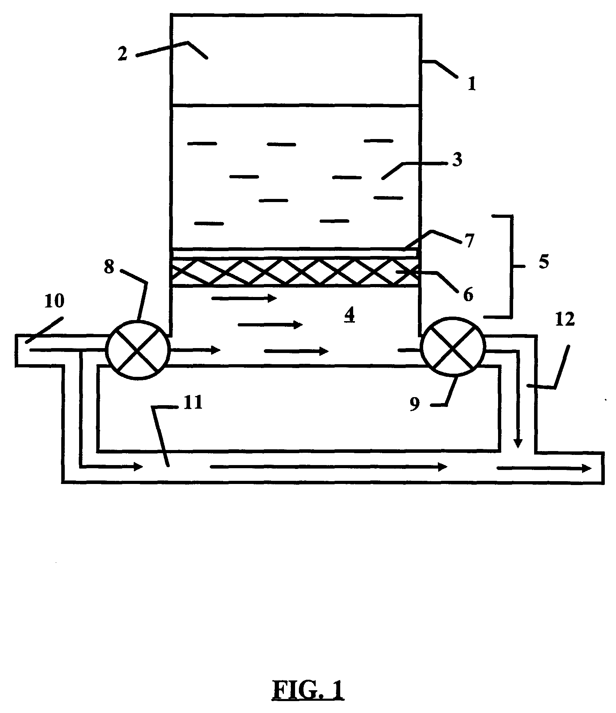

Referring now to Figure 1, a vaporiser comprises a vaporising chamber 1 which is divided into a liquid holding portion 2, in which an additive liquid such as an anaesthetic liquid 3 is contained, and a vapour receiving portion 4 by a porous wall 5. The porous wall 5 comprises a rigid support grating 6 a first face of which is directed towards the liquid 3 and is covered with a microporous membrane 7 having pores sized to prevent the passage of the liquid 3 through the wall 5 while allowing vapour to pass into the vapour receiving portion 4 of the chamber 1. The vaporisation of the liquid 3 is thus substantially independent of the level of the liquid 3. Additionally, by making the grating 6 of a thermally conducting material heat may be supplied to or taken away from it to thereby control the temperature of the liquid 3, at least near the grating 6. This may be used, for example, to supply heat to compensate for heat loss as the liquid 3 vaporises or to maintain the temperature of the liquid 3 below ambient in order to ensure that that the liquid 3 doesn't boil.

A valve controlled carrier gas inlet 8 and a valve controlled outlet 9 are disposed in the vapour receiving portion 4, opposing one another so as to define a carrier gas flow path which will direct a carrier gas through the vapour receiving portion 4 across a second face of the wall 5, opposing the first face, to collect the liquid vapour. Thus the concentration of anaesthetic vapour in the carrier gas 5 may be controlled simply by controlling the flow of gas through the vapour receiving portion 4.

A carrier gas line 10 is also provided with two branches 11,12 arranged in a manner common in the art. One branch 11 bypasses the vaporising chamber 1 and the other connects serially to the inlet 8 and to the outlet 9 of the chamber 1 and rejoins the bypass branch 11 after the outlet 9.

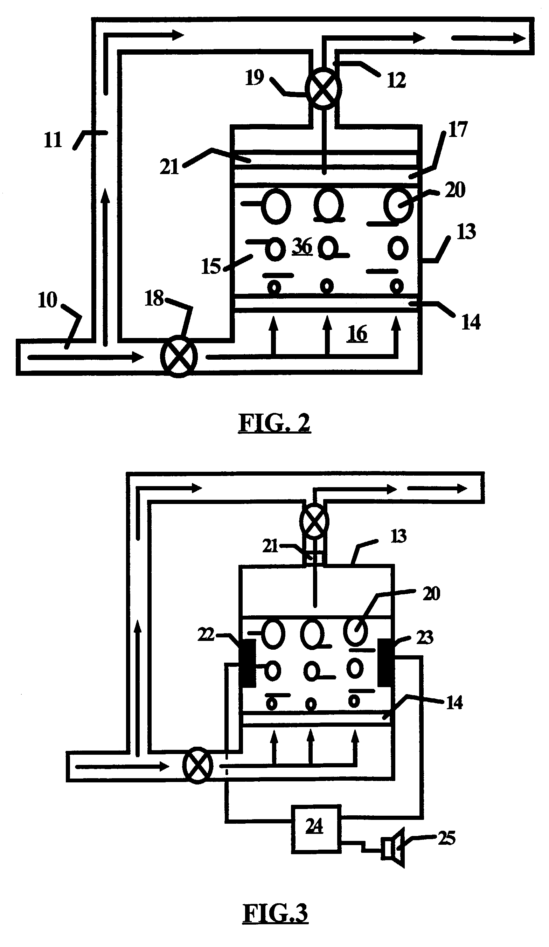

A second embodiment of the vaporiser of the present invention is shown in Figure 2. Here a vaporising chamber 13 is provided with a rigid porous wall 14 comprising a ceramic material in substitution for the microporous membrane 7 and support grating 6 arrangement shown in Figure 1 and having pores sized to exclude the passage of liquid therethrough. The wall 14 provides a support for a liquid additive 15, such as a liquid anaesthetic, contained within the chamber 13 and divides the chamber 13 into a liquid reservoir 36 and a carrier gas chamber 16 beneath the liquid 15 of the liquid reservoir 36. The chamber 13 is also provided with a vapour receiving portion 17 into which the liquid 15 may vaporise. A valve controlled carrier gas inlet 18 is provided to feed carrier gas into the carrier gas chamber 16 and together with a valve controlled outlet 19, sited in the vapour receiving portion 17 of the vaporisation chamber 13, defines a carrier gas flow path that directs carrier gas through the liquid anaesthetic 15. As carrier gas passes through the wall 14 bubbles 20 are formed in the liquid 15. These bubbles 20 originate across all of the porous wall 14 at the very bottom of the liquid 15 and burst when they reach the upper surface of the liquid 15 and a carrier gas saturated with anaesthetic vapour passes through the outlet 19. A further porous element 21 may be disposed within the vapour receiving portion 17 of the chamber 13 so as to prevent any liquid droplets from flowing through the outlet 19.

As with the embodiment of Figure 1, carrier gas line 10 is also provided with two branches 11,12 arranged in a manner common in the art. One branch 11 bypasses the vaporising chamber 1 and the other connects serially to the inlet 18 and to the outlet 19 of the chamber 13 and rejoins the bypass branch 11 after the outlet 19.

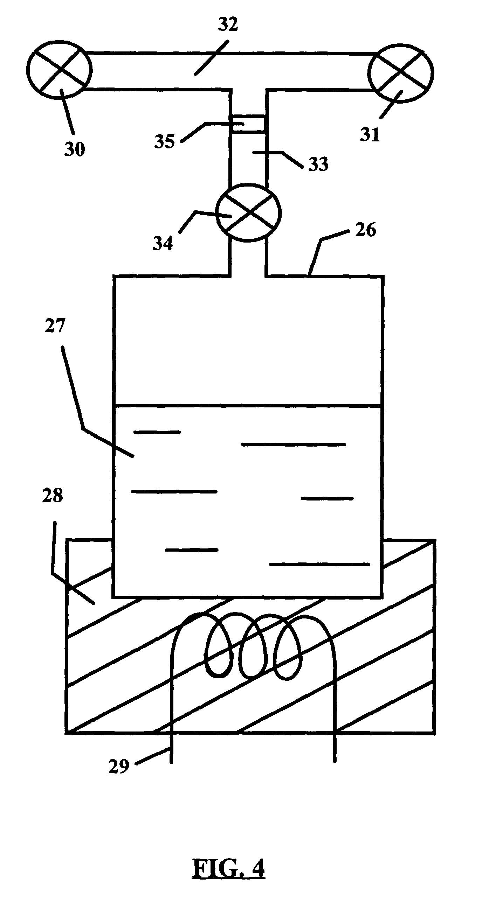

A modification to the embodiment of Figure 2 is shown in Figure 3 and corresponding components are identified by identical reference numbers. A light source 22 is positioned within the vaporising chamber 13 so as to be able to direct a beam of light through the liquid anaesthetic 15, in a direction not parallel (and preferably perpendicular) to the direction of carrier gas flow, for detection by a detector unit 23. A control unit 24 is operably connected to the source 22 to monitor and control its operation and is also operably connected to the detector 23 to receive a signal therefrom indicative of the level of incident light from the source 22. The control unit 24 is further adapted to analyse the received signal in order to determine whether or not light transmission between the source 22 and the detector 23 has been interrupted by the passage of a bubble 20. Light from the source 22 may be scattered by the passage of a bubble 20 through the light beam which will lead to a reduction in the intensity of the light detected by the detector 23. A comparison, within the control unit 24, of a received signal when no carrier gas is flowing (indicative of no bubbles being present) with a received signal when gas flows may be used to detect the presence, and even the number, of bubbles within the liquid 15. An output from the control unit 24 dependent on a detection having been made may be used to operate a sensible indicator unit, such as a loudspeaker 25. Thus the unit 25 then provides an operator with an indication that the vaporiser is working correctly.

A third embodiment of a vaporiser according to the present invention is shown in Figure 4. Here a vaporising chamber 26, sized to accommodate a liquid anaesthetic for vaporisation 27 and to provide a space above the liquid 26 into which vapour can be received, is in thermal contact with a thermally conductive block 28 and heater 29 arrangement. Vaporisation of the liquid 27 is then controlled by varying the heat supplied from the arrangement 28,29 to the chamber 26. A valve controlled carrier gas inlet 30 and an outlet 31 are arranged at opposite ends of a conduit 32 to define a carrier gas flow path lying entirely outside the vaporising chamber 26. A second conduit 33 is included in the vaporiser to provide a vapour flow path from the chamber 26 to the carrier gas conduit 32. A valve 34 is disposed in the vapour conduit 33 to regulate the flow of vapour into the carrier gas and hence the dose of anaesthetic. Also disposed within the vapour conduit 33 is a porous element 35 adapted to act as a barrier to the passage of liquid anaesthetic from the chamber 26 and into the carrier gas. Usefully this barrier 35 may be placed after the valve 34 to bar the passage of any liquid that may have condensed in the valve 34.

## Claims

1. A vaporiser comprising a vaporising chamber (1,13,26) for vaporising a liquid additive (3,15,27); a carrier gas inlet (8,18,30); a carrier gas outlet (9,19,31) co-operating with the inlet (8,18,30) to define opposite ends of a carrier gas flow path (12,32) which path is communicable with vapour of the vaporising chamber (1,13,26) **characterised in that** the vaporiser further comprises an element (5,14,35) of porous material having pores sized to exclude passage therethrough of the liquid additive (3,15,27) and disposed in the carrier gas flow path (12,32) as a barrier to the egress of the liquid additive (3,15,27)from the vaporiser.

2. A vaporiser as claimed in claim 1 **characterised in that** the element (5,14) is adapted to provide at least part of a liquid-tight boundary dividing the chamber (1,13) into a first portion (2,36) for holding the liquid additive and a second portion (4,16) from which the liquid additive (3,15) is excluded, the element (5,14) being arranged with a first surface exposed to the first portion (2,36) and an opposing second surface exposed to the second portion (4,16).

3. A vaporiser as claimed in claim 2 **characterised in that** the element (5,14) is adapted to provide at least part of a base wall of the first portion (2,36) supporting the liquid additive (3,15).

4. A vaporiser as claimed in any of the claims 2 or 3 **characterised in that** the inlet (8) and the outlet (9) are provided within the second portion (4) to define a carrier gas flow path to direct carrier gas through the second portion (4) of the chamber (1) over the second surface of the element (5).

5. A vaporiser as claimed in claims 3 **characterised in that** the inlet (18) and the outlet (19) are arranged to define a flow path to direct carrier gas from the second portion (16) through the element (14) and into the liquid additive (15) to generate carrier gas bubbles therein.

6. A vaporiser as claimed in claim 5 **characterised in that** there is provided a further element (21) of porous material having pores sized to exclude passage therethrough of the liquid additive (15) disposed in the carrier gas flow path to prevent egress of liquid additive through the outlet (19).

7. A vaporiser as claimed in any preceding claim **characterised in that** the element (5,14,35) of porous material comprises a microporous membrane (7) and a rigid grating (6) adapted to act as a support for the membrane (7).
